# FASCICULE DE BREVET EUROPEEN

(11) **EP 0 456 567 B1**
(45) Date de publication et mention de la délivrance du brevet: **07.01.1998**
(21) Numéro de dépôt: 91401181.2
(22) Date de dépôt: 06.05.1991
(51) Int. Cl.: C12Q 1/56

(54) **Procédé de détermination d'un activateur du plasminogène et de son inhibiteur**
Verfahren zur Bestimmung eines Plasminogenaktivators und sein Inhibitor
Procedure to determine a plasminogen activator and its inhibitor

(30) Priorité: 10.05.1990 FR 9005808
(43) Date de publication de la demande: 13.11.1991
(73) Titulaire: DIAGNOSTICA STAGO (société anonyme), F-92602 Asnières (FR)
(72) Inventeur: Contant, Geneviève épouse Pussard, F-92400 Courbevoie (FR); Martinoli, Jean-Luc, F-92390 Villeneuve la Garenne (FR); Quentin, Gerard, F-92700 Colombes (FR)
(74) Mandataire: Clisci, Serge

(56) Documents cités:
- EP-A- 0 020 780
- DE-A- 2 440 254
- HEMATOLOGY, vol. 7, 1988, pages 335-348, US; L.F. KRESS: "The action of snake venom metalloproteinases on plasma proteinase inhibitors"
- BIOCHIMICA ET BIOPHYSICA ACTA, vol. 615, 1980, pages 178-186, Elsevier/North- Holland Biomedical Press, NL; L.F. KRESS et al.: "Enzymatic inactivation of human antithrombin III, limited proteolysis of the inhibitor by sanke venom proteinases in the presence of heparin"
- CHEMICAL ABSTRACTS, vol. 107, no. 21, 23 novembre 1987, page 336, abrégé no. 193596h, Columbus, Ohio, US; J. AMIRAL et al.: "Laboratory assay for coagulation protein C", & THROMB. HEMORRH. DIS., PROC. INT. MEET. DANUBIAN LEAGUE THROMB. HAEMORRH. DIS., 4TH 1985 (PUB. 1986), 125-31

## Description

### DOMAINE DE L'INVENTION

La présente invention concerne une nouvelle solution technique pour la détermination ou le dosage d'un activateur du plasminogène choisi parmi l'ensemble constitué par les activateurs du plasminogène de type tissulaire et de type urokinase, d'une part, et d'un inhibiteur dudit activateur du plasminogène, d'autre part.

Elle vise le procédé pour la détermination d'un activateur du plasminogène et de son inhibiteur, qui comprend selon ladite nouvelle solution l'inhibition de deux protéines plasmatiques : les α₂-antiplasmine (α₂-AP) et α₂-macroglobuline (α₂-M).

### ART ANTERIEUR

Les activités des activateurs du plasminogène de type tissulaire (tPA) et de type urokinase (uPA) sont régulées par une substance inhibitrice dénommée inhibiteur d'activateur du plasminogène (PAI). On sait en particulier que les tPA et PAI jouent un rôle très important dans la régulation de la fibrinolyse et que les méthodes antérieurement connues pour leur dosage reposent sur le principe de la mesure de la plasmine, qui a été générée dans le système à partir du plasminogène, au moyen d'un substrat peptidique (fluorogène ou mieux chromogène) spécifique de la plasmine.

La fibrinolyse a pour but de restaurer la perméabilité vasculaire en dégradant les dépôts de fibrine susceptibles de se former sur les parois vasculaires. La fibrinolyse est assurée par la plasmine qui est un enzyme très puissant circulant dans le plasma sous sa forme inactive : le plasminogène.

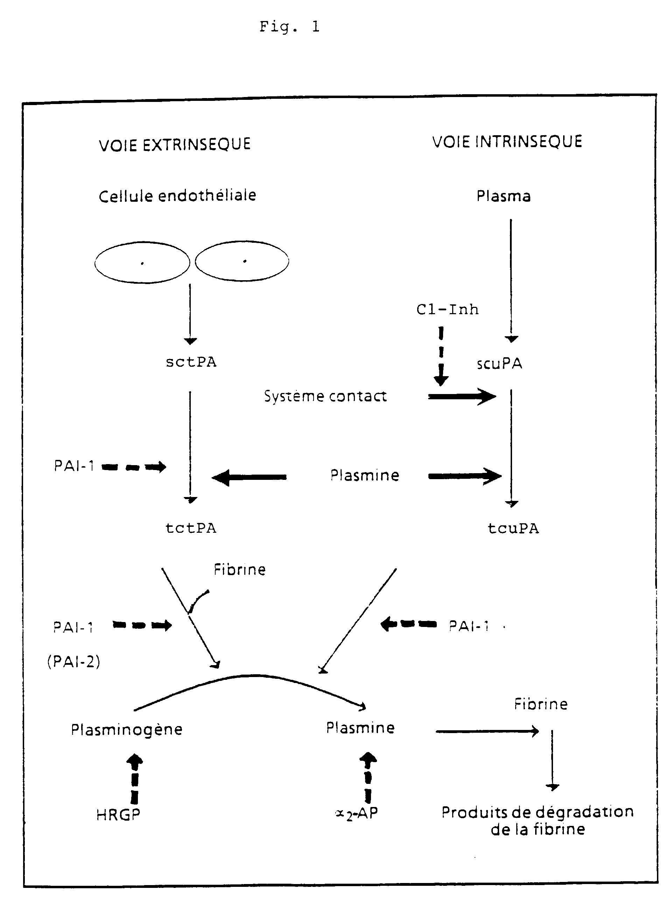

L'activation du plasminogène en plasmine peut se dérouler selon deux voies : une voie dite intrinsèque (ou endogène) et une voie dite extrinsèque (ou exogène). In vivo la fibrinolyse est perpétuellement régulée par un système d'inhibiteurs de telle façon que la fluidité sanguine soit toujours maintenue. Pour information, les mécanismes de la fibrinolyse ont été schématisés dans la figure 1 ci-après avec les interventions des activateurs et des inhibiteurs. Dans la figure 1, chaque flèche en trait épais continu désigne une activation, et chaque flèche en trait épais discontinu désigne une inhibition.

L'urokinase [également dénommée activateur du plasminogène de type urokinase (en abrégé : uPA)] intervient dans les mécanismes (voie intrinsèque) de la fibrinolyse d'abord sous la forme prourokinase [dénommée activateur du plasminogène de type urokinase de structure monocaténaire (en abrégé : scuPA)] puis sous la forme d'urokinase dite de haut poids moléculaire [également dénommée activateur du plasminogène de type urokinase de structure bicaténaire (en abrégé : tcuPA ou HMW-UK)]. Le tPA intervient dans les mécanismes (voie extrinsèque) de la fibrinolyse sous la forme dite sctPA [dénommée activateur du plasminogène de type tissulaire de structure monocaténaire (autre abréviation donnée dans la littérature : tPA-I)] et sous la forme dite tctPA [dénommée activateur du plasminogène de type tissulaire de structure bicaténaire (autre abréviation donnée dans la littérature : tPA-II)].

Il convient cependant de remarquer que, à l'état physiologique, le scuPA (voie intrinsèque) semble responsable, en grande partie, de l'activité fibrinolytique, et le tPA (voie extrinsèque) n'intervient que faiblement [95 % environ du tPA sont rapidement complexés par le PAI-1 dès sa libération; seuls restent 5 % environ du tPA sous forme libre et donc active dans le plasma]. En revanche, dès que l'organisme subit une agression quelconque, la voie extrinsèque joue alors un rôle prépondérant et essentiel pour pallier la formation excessive de plasmine.

Le tPA est principalement libéré des cellules endothéliales. Il peut être extrait de différents tissus d'origine animale (ovaire et coeur de porc) ou humaine (utérus). Toutefois, comme la quantité de tPA purifié extraite de ces tissus est infime, la source principale de tPA purifié que l'on préfère provient de la culture cellulaire de mélanomes humains (BOWES). Grâce à cette culture cellulaire qui sécrète beaucoup de tPA, on a pu obtenir le tPA purifié, estimer son poids moléculaire, déterminer sa structure biochimique et caractériser ses activités physiologiques. Le tPA est une sérine protéase ayant un poids moléculaire moyen d'environ 70 000 daltons, capable de transformer le plasminogène en plasmine. La présence de fibrine permet une activation optimale du plasminogène par le tPA.

L'inhibiteur d'activateur du plasminogène [en abrégé :PAI] intervient dans la régulation des activateurs du plasminogène. On connaît essentiellement quatre formes de PAI, à savoir le PAI-1 (inhibiteur 1 des activateurs du plasminogène), le PAI-2 (inhibiteur 2 des activateurs du plasminogène), le PAI-3 (inhibiteur 3 des activateurs du plasminogène) et la protéase nexine [voir Thrombosis and Haemostasis 56 (No 3), pages 415-416, (1986)].

Les inhibiteurs du tPA, qui sont également désignés sous le nom d'anti-activateurs, sont principalement de deux types :
le PAI-1 qui est présent dans le plasma et les granules alpha des plaquettes, et qui réagit aussi bien avec le tPA que l'urokinase pour donner des complexes inactifs : (tPA-PAI-1) et (uPA-PAI-1); comme la vitesse de complexation est très élevée, le fait qu'il existe du PAI-1 libre dans un plasma, implique qu'il ne peut pas il y avoir dans ce plasma de tPA libre actif; et,
le PAI-2 qui est d'origine probablement macrophagique, qui est à sa concentration maximale chez la femme enceinte au 3è trimestre de la grossesse, et qui, s'il forme des complexes inactifs avec le tPA, possède une affinité plus grande vis-à-vis de l'uPA; [voir E.D. SPRENGERS et al., Blood 69, (No 2), pages 381-387 (1987)].

En d'autres termes, on sait que les activités des activateurs du plasminogène de type tissulaire et urokinase sont régulées par au moins deux inhibiteurs spécifiques de type endothélial (PAI-1) et placentaire (PAI-2) et que les couples tPA/PAI et uPA/PAI ont un rôle essentiel dans la régulation de la fibrinolyse.

On sait par ailleurs que l'activité antiplasminique du plasma, qui est très importante, est principalement localisée au niveau des α₁-globulines et α₂-globulines. On connaît six protéines plasmatiques capables d'inhiber la plasmine en système purifié; ce sont :
- l'α₁-antitrypsine,
- l'inter-α-antitrypsine,
- l'inhibiteur de la C1 estérase (en abrégé C1-Inh),
- l'antithrombine III (en abrégé AT III),
- l'α₂-macroglobuline (en abrégé α₂-M), et
- l'α₂-antiplasmine (en abrégé α₂-AP).

En présence de concentrations plasmatiques normales en α₂-AP et α₂-M, les autres inhibiteurs de la plasmine interviennent peu dans l'inactivation de la plasmine. Le rôle de l'α₂-M est d'inhiber la plasmine en excès quand il n'y a plus d' α₂-AP libre. Ces inhibiteurs, principalement α₂-AP et α₂-M interfèrent de façon significative dans les dosages de PAI [voir l'article de G. CONTANT et al. Thrombosis Research 56, pages 377-386, (1989) et l'exposé de W. KAUSEL et al. présenté lors du Congrès de la Ligue Méditerranéenne de Lutte contre la Thrombose de 1988 à Athènes et intitulé : "Relative Contribution of Known Plasminogen Activator Inhibitors (PAIs) to the tPA and uPA Inhibitory Capacity of Plasma"].

Les méthodes qui ont déjà été préconisées, pour le dosage de tPA et PAI sont schématisées par les diagrammes A et B qui suivent. Elle reposent sur la mesure de la plasmine générée dans le système au moyen d'un substrat spécifique de la plasmine.

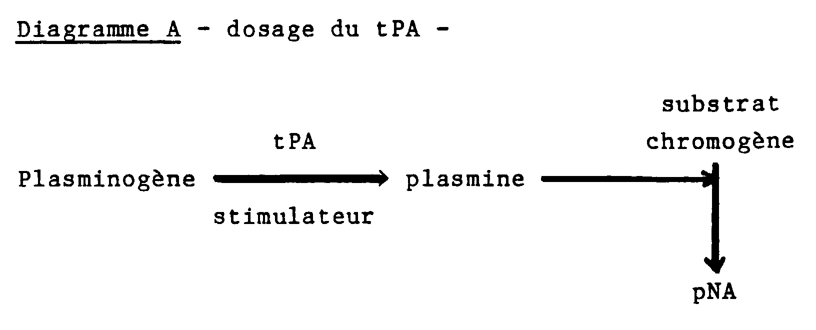
Diagramme A - dosage du tPA -

substrat
tPA chromogène
Plasminogène plasmine
stimulateur
pNA

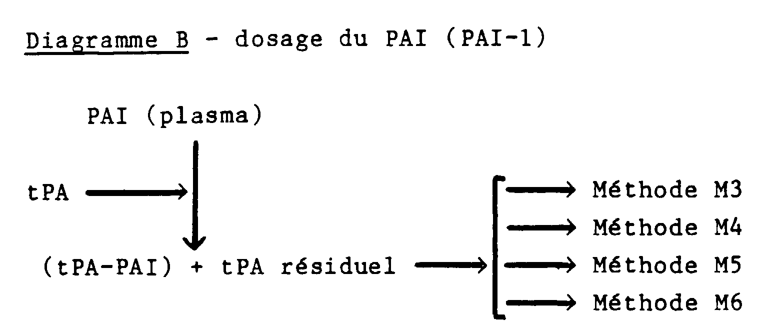
Diagramme B - dosage du PAI (PAI-1)

PAI (plasma)

tPA Méthode M3
Méthode M4
(tPA-PAI) + tPA résiduel Méthode M5
Méthode M6

On sait en particulier que les méthodes de dosage de l'activité biologique du tPA ont été préconisées dans le passé en phase solide et en phase liquide.
En phase solide (méthode M1), on connaît une technique fondée sur la fixation d'un réseau de fibrine à la surface de cupules d'une microplaque; après fixation de la source de tPA, des lavages sont effectuées afin d'éliminer la plupart des facteurs gênant la génération de plasmine. Cette technique présente des avantages :
- utilisation de toute source de tPA, comme le plasma,
- l'activité mesurée est spécifique du tPA et indépendante de la prourokinase [en abrégé : scuPA] et de l'urokinase [en abrégé : tcuPA].
Voir à cet effet les modalités de la méthode M1 décrite par E. ANGLES-CANO et al., "A Spectrophotometric Solid-Phase Fibrin-Tissue Plasminogen Activator Activity Assay (SOFIA-tPA) for High Fibrin Affinity Tissue Plasminogen Activators" Anal. Biochem., 153, pages 201-210, (1986). En phase liquide (méthode M2), on connait une technique selon laquelle, à la source de tPA à doser, on ajoute du plasminogène en excès et des fragments de fibrinogène solubles comme stimulateur. La plasmine en excès générée coupe le substrat spécifique de la plasmine [par exemple l'un des substrats CBS 30.41 (H-D-Abu-L-CHA-L-Lys-pNA) ou CBS 33.08 (H-D-Nle-L-CHA-L-Arg-pNA) commercialisés par la société DIAGNOSTICA STAGO] et libère le pNA détectable (notamment à 405 nm). La source de tPA peut être soit du tPA purifié (extrait de mélanome comme indiqué ci-dessus) que l'on utilise pour l'étalonnage, soit des euglobulines plasmatiques (dilution 1/10 et à pH 5,9), c'est-à-dire une source de tPA dépourvue d'inhibiteurs de la plasmine (surtout l'α₂-AP) qui gêneraient la deuxième étape de la réaction.

La méthode M2 est illustrée par la notice du test de dosage "STACHROM PA" de la société DIAGNOSTICA STAGO (référence No 0822 du catalogue de cette société).

L'activité mesurée est particulièrement liée au tPA grâce aux fragments de fibrinogène, mais elle est amplifiée par les autres voies d'activation (Facteur XII, prourokinase, urokinase). Pour que la mesure de l'activité soit spécifique du tPA, il est nécessaire d'ajouter des anticorps anti-tPA.

On connaît également une autre méthode (méthode M7) qui consiste à séparer le tPA libre par adsorption sur colonne de lysine-sépharose et qui a été décrite par E.GYZANDER et al., Thromb. Res., 35, pages 547-548, (1984).

Les méthodes applicables au dosage du PAI sont également applicables au dosage du tPA, dès lors qu'elles reposent sur la détermination du tPA résiduel après complexation du PAI contenu dans le plasma à tester. Elles comprennent notamment la méthode M3 décrite par C.KORNINGER et al., Thromb. Haem., 46, pages 622-665, (1981) et 52, pages 127-130, (1984) qui met en oeuvre des euglobulines; la méthode M4 décrite par J.CHMIELEWSKA et al., Thromb. Res., 31, pages 427-436, (1983) qui fait appel à du plasma acidifié; la méthode M5 décrite par J.H. VERREIJEN et al., Thromb. Haem., 48, pages 266-269, (1982) qui fait appel à la précipitation des euglobulines sur plasma pur ou dilué; et la méthode M6 décrite dans l'article de G. CONTANT et al. précité et utilisée dans la trousse de dosage commercialisée sous la nomenclature "STACHROM® PAI" (référence No 0824) par la société DIAGNOSTICA STAGO. Pour que la technique de G. CONTANT et al., précitée soit spécifique, il est important de diluer l'échantillon à tester dans du plasma déplété (i.e. appauvri ou épuisé) en PAI, l'interférence des inhibiteurs de la plasmine (autres que α₂-AP et α₂-M), apportés par le plasma devient alors négligeable.

Le principal inconvénient des méthodes précitées pour doser le tPA et/ou le PAI réside dans la nécessité de séparer le tPA libre des complexes tPA-PAI et uPA-PAI et d'empêcher l'action des inhibiteurs de la plasmine (plus particulièrement α₂-AP et α₂-M) par
- acidification du plasma (voir méthode M4),
- précipitation des euglobulines (voir méthode M5), ou
- adsorption sur colonne (voir méthode M7).

En bref, les méthodes antérieurement connues M1-M7 sont longues et délicates à mettre en oeuvre et sont susceptibles d'être déficientes en ce qui concerne la reproductibilité et/ou la sensibilité des dosages.

On connaît de EP-A-0 020 780 un extrait de venin de serpents appartenant à la famille des Trimesurus qui a une activité fibrinolytique. Cet extrait intervient en tant qu'activateur du plasminogène lors de la génération de la plasmine (voir EP-A-O 020 780, page 6 lignes 11 et 15-18). En revanche, le matériau métalloprotéinasique utilisé selon l'invention n'a aucune action sur le fibrinogène (voir exemple 4 ci-après).

Par ailleurs, comme on connaît l'inactivation des α-antiplasmines par oxydation notamment de l'article de D.JOHNSON et al., J.Biol.Chem., 254, pages 4022-4026, (1979), du résumé No 01358 de la communication de D. LAWRENCE et al.,(congrès "Thrombosis and Haemostasis" de 1985 à San Diego) et de l'article de D. LAWRENCE et al., Biochem., 25, pages 6351-6355, (1986) au moyen de chloramine T, on sait que l'on a proposé une nouvelle méthode (méthode M8) qui consiste à ajouter un oxydant spécifique des groupements méthionine, tel que la chloramine T, pour inhiber l'action de l'α₂-AP. [Voir à cet effet les articles de T.W.STIEF et al., Thromb. Res., 49, pages 581-589, (1988), 50, pages 559-573 (1988), 56, pages 213-220, (1989), la demande de brevet européen publiée EP-A-O 297 597, et la notice de la trousse de dosage commercialisée par la société BEHRING DIAGNOSTIKA sous la nomenclature "PAI-TEST" intitulée "FUNKTIONELLE BESTIMMUNG DES PLASMINOGEN-AKTIVATOR-INHIBITORS" (novembre 1988)]. Comme la chloramine T et les autres moyens oxydants sont des produits de manipulation délicate, il est apparu un besoin d'une nouvelle solution technique pour la détermination des activateurs du plasminogène de type tissulaire et de type urokinase, d'une part, et de leurs inhibiteurs, d'autre part, faisant appel à des moyens différents de ceux préconisés auparavant.

### BUT DE L'INVENTION

La nouvelle solution technique que l'on préconise selon l'invention pour la détermination d'un activateur du plasminogène choisi parmi l'ensemble constitué par les activateurs du plasminogène de type tissulaire et de type urokinase, d'une part, et d'un inhibiteur dudit activateur du plasminogène, d'autre part, repose sur l'utilisation d'un nouveau moyen inhibant ou annihilant les inhibiteurs de la plasmine, α₂-AP et/ou α₂-M.

Plus précisément, la présente invention repose sur la découverte surprenante que des venins de serpent contenant un matériau métalloprotéinasique inhibant les α₂-AP et/ou α₂-M et décrits dans l'article de L.F.KRESS intitulé "The Action of Snake Venom Metalloproteinases on Plasma Proteinase Inhibitors" et publié dans Hematology, 7, pages 335-348, (1988), ne dégradent ni ne détruisent les activateurs du plasminogène de type tissulaire et de type urokinase, d'une part, et leurs inhibiteurs PAI, d'autre part.

La nouvelle solution technique selon l'invention permet de pallier les inconvénients précités des méthodes de dosage antérieures sus-visées.

Selon un premier aspect de l'invention, on propose une nouvelle solution technique pour la détermination des activateurs du plasminogène et de leurs inhibiteurs qui met en oeuvre au moins un matériau métalloprotéinasique.

Selon un second aspect de l'invention, on préconise un procédé pour la détermination d'activateurs du plasminogène de type tissulaire (tPA) et de leurs inhibiteurs (PAI) dans lequel on utilise un matériau métalloprotéinasique.

Selon un autre aspect de l'invention, on préconise un nécessaire, trousse ou kit de dosage comprenant en tant que réactif au moins un matériau métalloprotéinasique.

### OBJET DE L'INVENTION

On propose selon l'invention un nouveau procédé pour la détermination dans le plasma (i) d'un activateur du plasminogène choisi parmi l'ensemble constitué par les activateurs du plasminogène de type tissulaire (tPA) et les activateurs du plasminogène de type urokinase (uPA), et (ii) d'un inhibiteur (PAI) dudit activateur du plasminogène, ledit procédé, qui comporte l'inhibition des protéines plasmatiques α₂-antiplasmine et/ou α₂-macroglobuline au moyen d'une substance choisie parmi l'ensemble constitué par les matériaux métalloprotéinasiques, d'une part, et la transformation du plasminogène en plasmine puis le dosage de la plasmine résultant de ladite transformation, d'autre part, étant caractérisé en ce qu'il comprend (1) l'incorporation du matériau métalloprotéinasique dans le plasma à étudier, (2) l'ajout dans le milieu résultant d'un moyen stimulant la transformation du plasminogène en plasmine, puis (3) le dosage de la plasmine qui résulte de ladite transformation.

On propose également un nécessaire pour la détermination d'un activateur du plasminogène de type tissulaire ou de type urokinase, d'une part, ou d'un inhibiteur d'activateur du plasminogène, d'autre part, caractérisé en ce qu'il comprend (A) au moins un matériau métalloprotéinasique choisi parmi l'ensemble constitué par les venins de Bitis arietans, Crotalus basiliscus et Lachesis muta, et (B) au moins un activateur du plasminogène choisi parmi l'ensemble constitué par les activateurs du plasminogène de type tissulaire (tPA) et les activateurs du plasminogène de type urokinase (uPA).

### ABREVIATIONS

Par commodité, les abréviations suivantes ont été utilisées dans la présente description, à savoir :
a) en ce qui concerne la fibrinolyse :
   - α₂-AP =: α₂-antiplasmine
   - C1-Inh =: inhibiteur de la C1 estérase
   - α₂-M =: α₂-macroglobuline
   - tPA =: activateur du plasminogène de type tissulaire (en anglais : "tissue plasminogen activator")
   - sctPA =: activateur du plasminogène de type tissulaire de structure monocaténaire (en anglais : "single-chain tissue plasminogen activator"); autre nomenclature : tPA-I
   - tctPA =: activateur du plasminogène de type tissulaire de structure bicaténaire (en anglais : "two-chain tissue plasminogen activator); autre nomenclature : tPA-II
   - uPA =: activateur du plasminogène de type urokinase (en anglais : "urokinase plasminogen activator"); autres nomenclatures : urokinase ou activateur du plasminogène de type urinaire
   - scuPA =: activateur du plasminogène de type urokinase de structure monocaténaire (en anglais : "single-chain urokinase plasminogen activator"); autre nomenclature : prourokinase
   - tcuPA =: activateur du plasminogène de type urokinase de structure bicaténaire (en anglais : "two-chain urokinase plasminogen activator"); autres nomenclatures : urokinase de haut poids moléculaire ou HMW-UK
   - PAI =: inhibiteur d'activateur du plasminogène
   - PAI-1 =: inhibiteur 1 des activateurs du plasminogène, substance formant des complexes (tPA-PAI-1) et (uPA-PAI-1) avec tPA et respectivement uPA
   - PAI-2 =: inhibiteur 2 des activateurs du plasminogène, substance formant des complexes avec tPA et uPA
   - PAI-3 =: inhibiteur 3 des activateurs du plasminogène, substance susceptible de former des complexes avec uPA
   - UK =: urokinase (autre nomenclature de uPA).
b) en ce qui concerne les substrats peptidiques :
   - Arg =: arginyle
   - Abu =: 2-aminobutyryle
   - CHA =: 3-cyclohexylalanyle
   - CHT =: 3-(4-hydroxycyclohexyl)alanyle
   - Glu =: glutaminyle
   - Gly =: glycyle
   - Hyp =: hydroxyprolyle (3Hyp ou 4Hyp)
   - 3Hyp =: 3-hydroxyprolyle (ou 3-hydroxy-2-pyrrolidinecarbonyle)
   - 4Hyp =: 4-hydroxyprolyle (ou 4-hydroxy-2-pyrrolidinecarbonyle)
   - Leu =: leucyle
   - Lys =: lysyle
   - Nle =: norleucyle
   - Nva =: norvalyle
   - Phe =: phénylalanyle
   - Phg =: phénylglycyle
   - Pip =: pipécolinoyle
   - Pro =: prolyle
   - Pyr =: pyroglutaminyle (ou 2-pyrrolidone-5-carbonyle)
   - Tyr =: tyrosyle
   - Val =: valyle
c) en ce qui concerne les autres abréviations :
   - AMCHA =: acide tranexamique, de nomenclature systématique : acide 4-(aminométhyl)-cyclohexane-carboxylique
   - BSA =: albumine bovine sérique
   - Bzl =: benzyle
   - EACA =: acide ε-aminocaproïque
   - EM =: éthyloxymalonyle (EtO-CO-CH₂-CO)
   - Et =: éthyle
   - Me =: méthyle
   - MM =: méthyloxymanolyne (MeO-CO-CH₂-CO)
   - OD =: densité optique
   - OtBu =: t-butyloxy
   - PEGₙ =: reste de polyéthylèneglycol de PM = n
   - PM =: poids moléculaire
   - pNA =: p-nitroanilino [ou (4-NO₂)C₆H₄NH]
   - RT =: température ambiante (15-20°C)

### DESCRIPTION DETAILLEE DE L'INVENTION

Par l'expression "matériau métalloprotéinasique" on entend selon l'invention une substance constituée par une ou plusieurs métalloprotéinases ou une substance renfermant au moins une métalloprotéinase. Le matériau métalloprotéinasique qui intervient selon l'invention a pour objet d'annihiler, d'inhiber ou détruire les protéases plasmatiques que sont les α₂-AP et/ou α₂-M.

Parmi les matériaux métalloprotéinasiques qui conviennent selon l'invention, on peut notamment citer les venins. Les venins contenant au moins une métalloprotéinase peuvent être utilisés soit tels que recueillis notamment de serpents ou d'insectes, soit purifiés. Parmi les venins de serpent que l'on peut utiliser, on peut notamment citer ceux obtenus d'animaux appartenant aux familles des vipéridés et des crotalidés.

Le matériau métalloprotéinasique préféré selon l'invention est choisi parmi les produits qui clivent l'α₂-antiplasmine de poids moléculaire de l'ordre de 68 000 daltons pour donner un peptide qui est essentiellement dépourvu d'activité α₂-AP et a un PM de l'ordre de 53 000 daltons.

Les matériaux métalloprotéinasiques que l'on préconise de préférence selon l'invention sont constitués par les venins de Bitis arietans, Crotalus basiliscus et Lachesis muta (ou Lachesis mutus).

On sait que les ω-aminoacides tels que l'AMCHA et l'EACA favorisent la transformation du plasminogène en plasmine selon deux mécanismes : induction d'un changement de conformation du Glu-plasminogène en Lys-plasminogène plus activable en plasmine, et interférence sur l'interaction de la plasmine avec l'α₂-AP permettant de diminuer l'effet inhibiteur de la plasmine par l'α₂-AP d'au moins 10 fois.

On vient de trouver de façon surprenante que lesdits ω-aminoacides potentialisent l'effet recherché du matériau métalloprotéinasique sur l'α₂-AP et l'α₂-M, lorsque l'on fait appel à un plasma non épuisé en α₂-AP, en présence ou en absence de PAI. En revanche, il n'y a pas de potentialisation de l'effet dudit matériau métalloprotéinasique lorsque l'on fait appel à un plasma épuisé en α₂-AP, dès lors que, dans ce cas, la génération de plasmine à partir du plasminogène est suffisamment importante avec ledit matériau métalloprotéinasique.

Dans le cas de la détermination ou du dosage de tPA, l'on recommande selon l'invention d'incorporer un stimulateur. Le stimulateur utilisable est ici un moyen classique de l'art antérieur, par exemple des monomères de fibrine commercialisés notamment sous le nom commercial de DESASIF® [des-AA-fibrinogène] par la société BIOPOOL, des fragments de fibrinogène (voir l'article de VERHEIJEN précité) et commercialisés notamment par la société DIAGNOSTICA STAGO dans sa trousse de dosage STACHROM® PA, ou une polylysine décrite notamment dans l'article de GYZANDER précité.

En pratique le procédé selon l'invention comprend, pour la détermination ou le dosage du tPA, les modalités selon lesquelles (a) l'on incorpore le matériau métalloprotéinasique dans le plasma à étudier dilué dans le tampon approprié, (b) incube à 37°C pendant 2 minutes, (c) ajoute un moyen stimulant la transformation du plasminogène en plasmine, (d) incube à 37°C pendant 150 minutes, (e) ajoute le substrat chromogène spécifique de la plasmine, puis (f) incube à 37°C pendant 5 minutes, la lecture de la densité optique correspondant à la libération du moyen chromogène étant ensuite effectuée notamment par comparaison avec un essai à blanc dans lequel le plasma à étudier additionné de matériau métalloprotéinasique du stade (a) est remplacé par un tampon additionné de matériau métalloprotéinasique.

Le procédé, pour la détermination ou le dosage du PAI, comprend les modalités selon lesquelles (a) l'on incorpore tPA ou uPA dans le plasma à étudier, (b) incube à 37°C pendant 5 minutes, (c) ajoute un mélange constitué par du plasminogène, un ω-aminoacide et ledit matériau métalloprotéinasique, (d) incube à 37°C pendant 3 minutes, (e) ajoute le substrat chromogène spécifique de la plasmine, puis (f) incube à 37°C pendant 3 minutes, la lecture de la densité optique correspondant à la libération du moyen chromogène étant ensuite effectuée notamment par comparaison avec un essai à blanc dans lequel au stade (c) l'activateur du plasminogène (tPA ou uPA) est remplacé par un tampon.

De façon avantageuse selon l'invention, l'on préconise pour la détermination ou le dosage de tPA d'utiliser une concentration de venin de Bitis arietans, Crotalus basiliscus ou Lachesis muta dans un tampon approprié supérieure ou égale à 0,025 mg/ml (correspondant à une teneur supérieure ou égale à 0,0125 mg/ml finale dans le mélange de l'essai). En pratique, la concentration maximale du venin dans le tampon sera inférieure ou égale à 1 mg/ml. De préférence, l'on utilisera plus avantageusement une concentration de venin de Bitis arietans, Crotalus basiliscus ou Lachesis muta dans le tampon de l'ordre de 0,05 mg/ml. Le tampon préféré sera un tampon phosphate de pH 7,0 à 9,0 et mieux un tampon phosphate de pH 7,5. Ledit tampon peut contenir de l'albumine bovine (BSA) pour favoriser la lyophilisation des réactifs devant être incorporés dans les trousses de dosage.

Pour le dosage ou la détermination de PAI, on préconise avantageusement d'utiliser une concentration de venin dans le plasma de 0,1 à 1 mg/ml, et de préférence une concentration de 0,24 mg/ml.

Selon le meilleur mode de mise en oeuvre de l'invention, pour la détermination ou le dosage du tPA, (a) l'on incorpore du venin de Bitis arietans, Crotalus basiliscus ou Lachesis muta dans le plasma à étudier dilué dans le tampon approprié, (b) incube à 37°C pendant 2 minutes, (c) ajoute le moyen stimulant la transformation du plasminogène en plasmine (notamment le produit commercialisé sous le nom de STIMUGEN par la société DIAGNOSTICA STAGO), (d) incube à 37°C pendant 150 minutes, (e) ajoute le substrat chromogène, puis (f) incube à 37°C pendant 5 minutes. On effectue ensuite la lecture de la libération du moyen chromogène, notamment à 405 nm, soit par une méthode statique dite "à point terminal" (en anglais :"end-point technique"), soit par une méthode cinétique dite "à deux points" (en anglais : "two-point technique") (en ajoutant dans ce cas de l'acide acétique). La lecture de OD (notamment par transmission) est contrôlée par un essai à blanc dans lequel le plasma à étudier additionné de venin de Bitis arietans, Crotalus basiliscus ou Lachesis muta est remplacé par le tampon additionné dudit venin.

En variante, le matériau métalloprotéinasique, ici le venin de Bitis arietans, Crotalus basiliscus ou Lachesis muta, peut être incorporé dans le système au stade (c) avec le moyen stimulant.

Toujours selon le meilleur mode de mise en oeuvre de l'invention, pour la détermination ou le dosage du PAI, (a) l'on incorpore tPA ou uPA dans le plasma à étudier, (b) incube à 37°C pendant 5 minutes, (c) ajoute un mélange constitué par du plasminogène, un ω-aminoacide (notamment AMCHA ou EACA) et du venin de Bitis arietans, Crotalus basiliscus ou Lachesis muta, (d) incube à 37°C pendant 3 minutes, (e) ajoute le substrat chromogène, puis (f) incube à 37°C pendant 3 minutes. On effectue ensuite la lecture de la libération du moyen chromogène, comme indiqué ci-dessus, notamment à 405 nm, par la méthode dite à point terminal ou en cinétique selon la méthode dite à deux points (en ajoutant dans ce cas de l'acide acétique). La lecture de OD (notamment par transmission) est également contrôlée par un essai à blanc dans lequel le tPA ou l'uPA est remplacé par un tampon.

Parmi les substrats chromogènes synthétiques spécifiques de la plasmine, l'on peut utiliser notamment l'un des composés suivants : 
H-D-Nva-L-CHA-L-Lys-pNA,
H-D-Abu-L-CHT-L-Lys-pNA,
 
H-D-Nle-L-CHA-L-Arg-pNA,
H-D-Phe-L-Pip-L-Arg-pNA,
H-D-Val-Leu-Lys-pNA,
MM-L-Phe-L-Arg-pNA,
MM-L-4Hyp-L-Arg-pNA,
MM-L-3Hyp-L-Arg-pNA,
MM-L-Tyr-L-Arg-pNA,
MM-L-Phg-L-Arg-pNA,
MM-L-Hyp-L-Lys-pNA,
MM-L-Pro-L-Lys-pNA,
MM-L-Tyr-L-Lys-pNA,
MM-L-Hyp(OtBu)-L-Arg-pNA,
EM-L-Pro-L-Arg-pNA,
EM-L-Tyr-L-Arg-pNA,
EM-L-Phe-L-Arg-pNA,
EM-L-Phg-L-Arg-pNA,
PEG200[CO-D-Glu(OBzl)-L-Pro-L-Arg-pNA]2,
PEG400[CO-D-Leu-Gly-L-Arg-pNA]2,
 (les deux derniers substrats de cette liste étant décrits dans la demande de brevet français No 90 01964 de la Demanderesse) ou un de leurs sels d'addition d'acide, notamment avec HCl, l'acide acétique ou l'acidetrifluoroacétique comme acide salifiant.

Le nécessaire, trousse ou kit de dosage selon l'invention, comprendra au moins un matériau métalloprotéinasique de préférence choisi parmi les venins de Bitis arietans, Crotalus basiliscus, Lachesis muta et leurs mélanges, et des échantillons tPA, uPA et/ou PAI purs.

La solution selon l'invention, qui met en oeuvre un matériau métalloprotéinasique pour inhiber les protéases plasmatiques α₂-AP et/ou α₂-M, convient pour le dosage des activateurs du plasminogène de type tissulaire et de type urokinase, et leurs inhibiteurs. La solution selon l'invention est plus particulièrement destinée au dosage ou à la détermination de tPA et de son inhibiteur, le PAI.

### EXEMPLE 1

### - Protocole de dosage de tPA -

On procède selon le diagramme A ci-dessus dans un tube à hémolyse en matière plastique maintenu à la température de 37°C, selon les modalités données dans le tableau I ci-après.

**TABLEAU I**

| DOSAGE DU tPA | | |
|---|---|---|
| Ingrédients | Essai | Blanc |
| Plasma dilué dans un tampon avec matériau métalloprotéinasique (a) | 200 µl | - |
| Tampon plus matériau métalloprotéinasique (a) | - | 200 µl |
| Incubation | 2 minutes | 2 minutes |
| Stimulant (b) | 200 µl | 200 µl |
| Incubation | 150 minutes | 150 minutes |
| Substrat (c) | 200 µl | 200 µl |
| Notes (a) venin de B.arietans, C.basiliscus ou L.muta (b) produit commercialisé sous le nom de STIMUGEN par la société DIAGNOSTICA STAGO (c) substrat chromogène spécifique de la plasmine Remarque La lecture est faite avec un spectrophotomètre pour apprécier la libération du groupement chromogène à 405 nm. | | |

### EXEMPLE 2

### - Dosage de PAI -

On procède selon le diagramme B ci-dessus dans un tube à hémolyse en matière plastique maintenu à la température de 37°C, selon les modalités données dans le tableau II ci-après.

**TABLEAU II**

| DOSAGE DU PAI | | |
|---|---|---|
| Ingrédients | Essai | Blanc |
| Plasma | 50 µl | 50 µl |
| uPA ou tPA | 200 µl | - |
| Tampon | - | 200 µl |
| Incubation | 5 minutes | 5 minutes |
| Plasminogène plus (a) plus (b) | 200 µl | 200 µl |
| Incubation | 3 minutes | 3 minutes |
| Substrat (c) | 200 µl | 200 µl |
| Incubation | 3 minutes | 3 minutes |
| Notes (a) venin de B.arietans, C.basiliscus ou L.muta (b) ω-aminoacide (AMCHA ou EACA) (c) substrat chromogène spécifique de la plasmine Remarque La lecture est faite avec un spectrophotomètre pour apprécier la libération du groupement chromogène à 405 nm. | | |

### EXEMPLE 3

### - Effet du matériau métalloprotéinasique sur α₂-AP -

On a mesuré la teneur en α₂-AP d'un plasma normal au moyen de la trousse de dosage "STACHROM α₂-AP" commercialisée par la société DIAGNOSTICA STAGO, en présence ou en l'absence d'un matériau métalloprotéinasique constitué par du venin de B.arietans, C.basiliscus ou L.muta, la teneur en venin étant à une concentration supérieure ou égale à 0,50 mg/ml de plasma en tampon de dilution (soit une teneur supérieure ou égale à 0,0125 mg/ml du mélange contenant de la plasmine).

L'inhibition de l'α₂-AP mesurée avec quatre lots différents de venin de B.arietans, quatre lots différents de C.basiliscus et quatre lots différents de L. muta est obtenue pour des concentrations allant de 0,5 à 1 g/ml de plasma (soit 0,025 à 0,050 g/l de tampon) de B.arietans, d'une part, et pour la concentration de 0,5 mg/ml de plasma (soit 0,025 mg/ml de tampon) de C.basiliscus ou de L.muta, d'autre part.

Quand on dose l'α₂-AP d'un plasma normal (teneur en α₂-AP dite de 100 %) en présence d'un desdits venins, on trouve une teneur (relative) en α₂-AP d'environ 25 % (20 à 30 %), ce qui démontre une inhibition de l'α₂-AP par le matériau métalloprotéinasique contenu dans le venin de B.arietans, C.basiliscus ou L.muta.

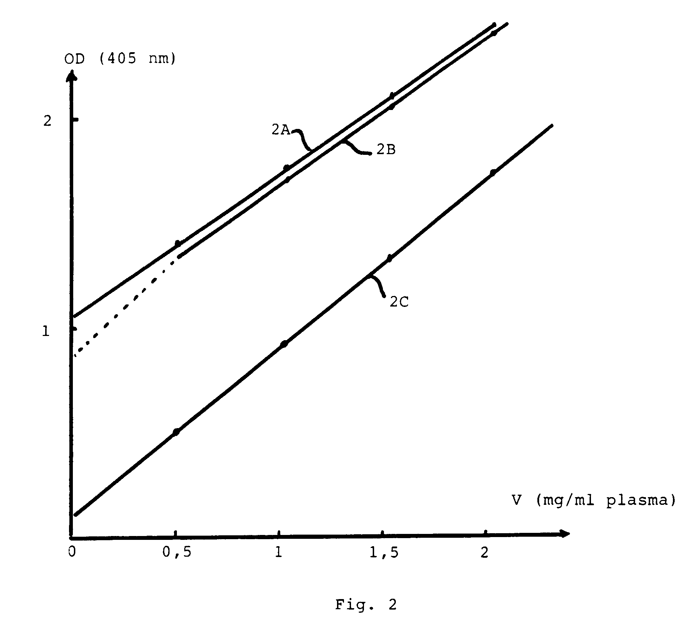

Les résultats consignés dans la figure 2 ci-après dans le système OD en ordonnées (à 405 nm)/V (venin de B.arietans) en mg/ml en abscisses confirment l'inhibition de l'α₂-AP par ledit venin, la courbe 2A correspondant à un échantillon contenant le tampon et de la plasmine, la courbe 2B correspondant à un échantillon contenant le plasma et de la plasmine, la courbe 2C correspondant à un échantillon contenant le tampon sans plasmine.

### EXEMPLE 4

### - Effet du matériau métalloprotéinasique sur le plasminogène et la plasmine -

On a observé que le matériau métalloprotéinasique (notamment le venin de B.arietans, C.basiliscus ou L. muta) n'a pas d'effet sur le plasminogène et qu'il n'a pas d'effet statistiquement significatif sur la plasmine. En particulier lorsque l'on ajoute le matériau métalloprotéinasique à de la plasmine puis incube à 37°C, on constate que le matériau métalloprotéinasique est capable d'hydrolyser les substrats synthétiques spécifiques de la plasmine [notamment les substrats CBS 30.41 et CBS 10.65, qui sont respectivement les H-D-Abu-L-CHA-L-Lys-pNA,AcOH et MM-Hyp-Arg-pNA,AcOH]. Cette hydrolyse est fonction de la concentration en venin; quand on utilise une concentration en venin relativement faible (0,0125 mg/ml de milieu), 95 % de la plasmine est active sur le substrat : l'effet du venin sur la plasmine est alors statistiquement non significatif.

### EXEMPLE 5

### - Effet du matériau métalloprotéinasique sur uPA et tPA -

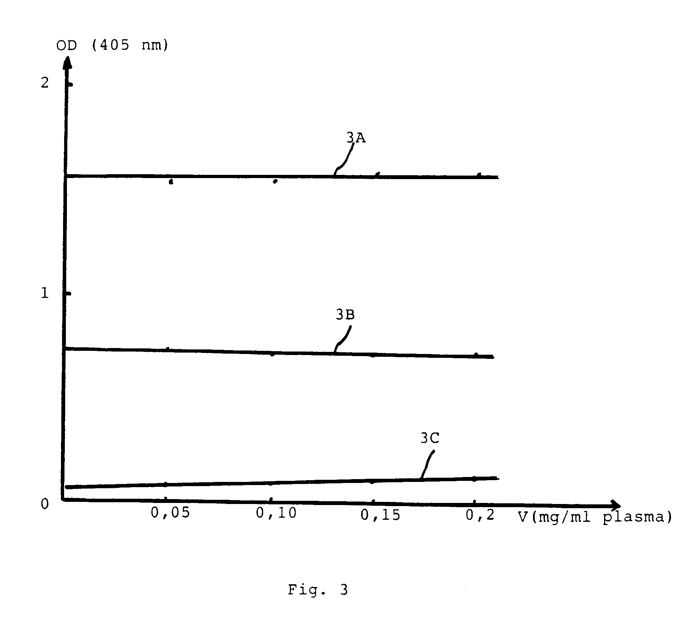

On a observé que l'activité de uPA (urokinase) directement mesurée sur un substrat spécifique n'est pas modifiée en présence de venin (voir figure 3).

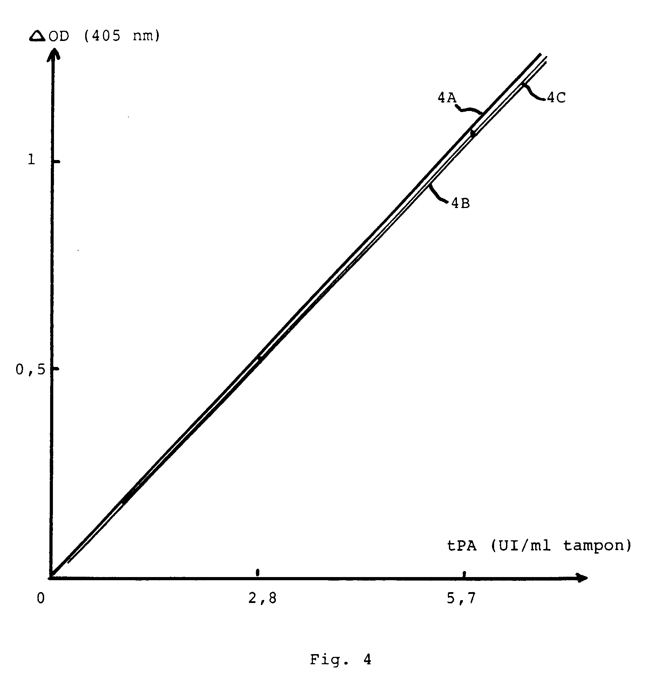

On a également observé que l'activité du tPA mesurée par STACHROM PA n'est pas modifiée en présence de venin (voir figure 4).

Dans la figure 3, selon le système OD en ordonnées (à 405 nm)/V (venin de B.arietans) en mg/ml en abscisses, les courbes 3A (uPA à 100 UI/ml), 3B (uPA à 40 UI/ml) et 3C (tampon seul) sont approximativement des droites.

Dans la figure 4, selon le système OD en ordonnées (à 405 nm)/tPA (dans le tampon) en UI/ml en abscisses, les courbes 4A (tampon contenant 0,24 mg/ml de venin de B.arietans), 4B (tampon contenant 0,50 mg/ml de venin de B.arietans) et 4C (tampon sans venin) sont des droites pratiquement confondues.

### EXEMPLE 6

### - Effet du matériau métalloprotéinasique sur PAI -

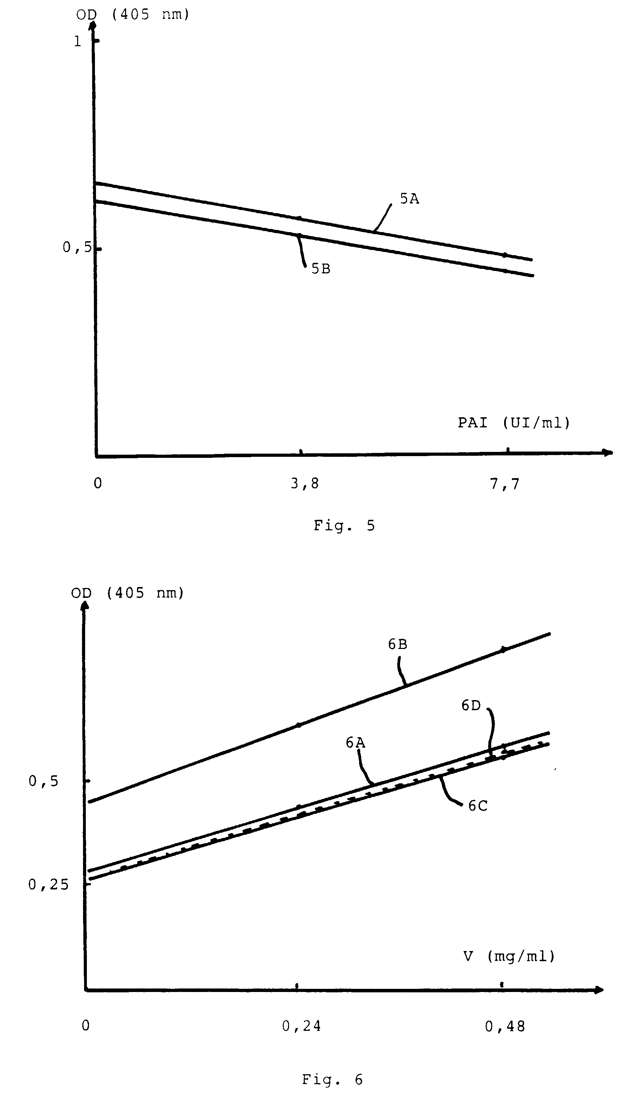

On a observé que, lorsqu'un plasma riche en PAI est incubé en présence d'un matériau métalloprotéinasique (venin de B.arietans, C.basiliscus ou L.muta), l'activité du PAI telle que dosée est identique à celle trouvée dans ledit plasma en l'absence dudit matériau métalloprotéinasique (voir figure 5).

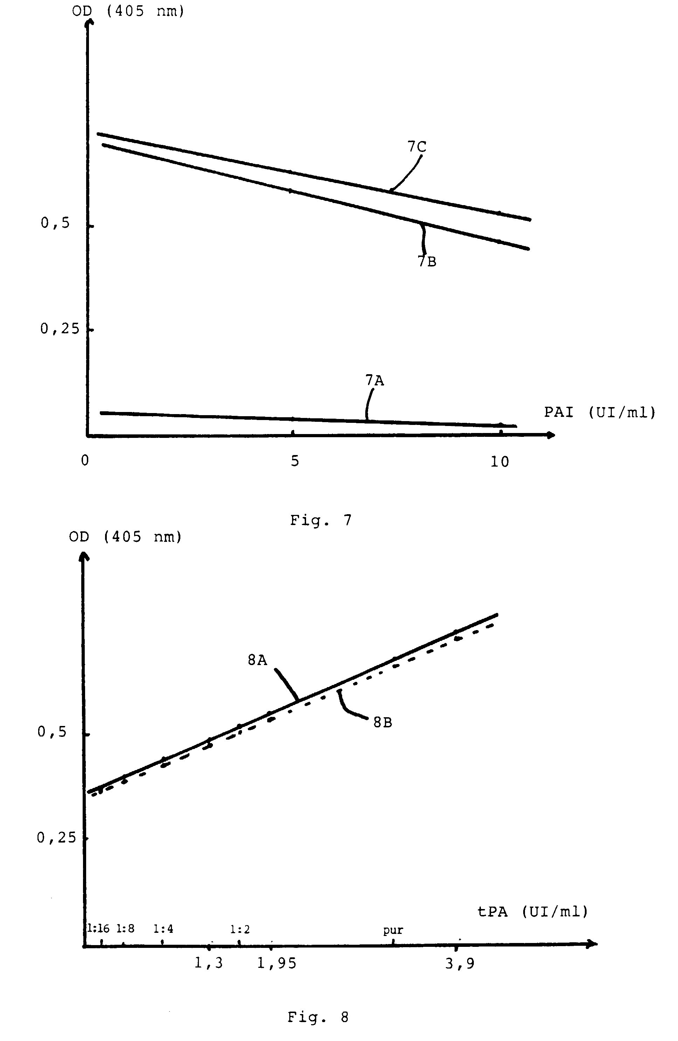

On a également observé que, lorsque l'on ajoute du tPA purifié au mélange plasma-matériau métalloprotéinasique (venin de B.arietans, C.basiliscus ou L.muta), le tPA ajouté est totalement inhibé par le PAI du plasma en l'absence et en présence dudit matériau métalloprotéinasique (voir figure 6).

L'ensemble de ces résultats met clairement en évidence que, selon l'invention, le matériau métalloprotéinasique n'inhibe pas le PAI.

Dans la figure 5, selon le système OD en ordonnées (à 405 nm)/PAI en UI/ml en abscisses, les courbes 5A (PAI en l'absence de venin de B.arietans) et 5B (PAI en présence de venin de B.arietans) sont des droites parallèles.

Dans la figure 6, selon le système OD en ordonnées (à 405 nm)/venin (de B.arietans) en mg/ml en abscisses, le tPA a été ajouté à un plasma PO (teneur en PAI de O UI/ml) déplété en PAI ou à un plasma P1 (teneur en PAI de 7,7 UI/ml) riche en PAI, en présence de concentrations croissantes en venin de O à 0,50 mg/ml. La courbe 6A (plasma PO sans tPA) est une droite qui est approximativement parallèle à la droite 6B (tampon PO plus tPA 5,2 UI/ml). Les droites 6C (plasma P1 sans tPA) et 6D (plasma P1 plus tPA 5,2 UI/ml) sont identiques; ceci indique que le tPA ajouté au plasma (P1) riche en PAI est inhibé en l'absence ou en présence de venin.

### EXEMPLE 7

### - Effet du matériau métalloprotéinasique sur les complexes uPA-PAI -

On a apprécié l'éventuelle interaction du matériau métalloprotéinasique (venin de B.arietans brut, tel que recueilli et fourni par les fermes d'élevage ) sur les complexes uPA-PAI formés par incubation avec l'uPA (urokinase) à 37°C pendant 0,25 h d'un plasma riche en PAI (plasma P2 contenant du PAI à la concentration de 10 UI/ml) par comparaison avec le plasma PO épuisé en PAI utilisé à l'exemple 6.

Les résultats obtenus avec le venin de B.arietans ont été consignés dans les tableaux III et IV ci-après. Lesdits résultats montrent qu'il n'y a pas de formation de complexe dans le plasma PO car il n'y a pas de PAI : on retrouve donc tout l'uPA résiduel; comme le plasma P2 contient 10 UI/ml de PAI, il y a formation de complexes avec l'uPA et l'on ne retrouve qu'une partie de l'uPA ajouté. Ces résultats montrent également que le venin n'a pas d'effet sur la dissociation des complexes uPA-PAI et tPA-PAI.

### EXEMPLE 8

### - Effet du matériau métalloprotéinasique et de l'AMCHA sur la transformation du plasminogène en plasmine par l'uPA ou le tPA -

En premier lieu, on a vérifié que l'AMCHA, oméga-aminoacide de référence, augmente la génération de plasmine dans le dosage de PAI en présence d'urokinase (voir les résultats donnés dans la figure 7).

Dans la figure 7, selon le système OD en ordonnées (à 405 nm)/PAI en UI/ml en abscisses, les courbes obtenues : 7A (sans AMCHA), 7B (AMCHA à 1,3 mmoles/l dans le réactif) et 7C (AMCHA à 2,6 mmoles/l dans le réactif), sont des droites et montrent que l'AMCHA augmente effectivement la transformation du plasminogène en plasmine.

En deuxième lieu, on a voulu vérifier que l'AMCHA diminue l'effet de l'α₂-AP d'un plasma normal (c'est-à-dire un plasma ayant une teneur en α₂-AP de 100 %). Selon la méthode "STACHROM α₂-AP" précitée, on a constaté que le taux d'α₂-AP est en moyenne de 30 % à une concentration de 0,88 mmole/l de tampon de dilution.

En troisième lieu, comme le venin brut (venin de B.arietans) est capable d'inhiber l'α₂-AP du plasma, soit directement soit en interférant sur l'interaction α₂-AP - plasmine, on a recherché si cet effet est potentialisé par l'AMCHA selon les modalités suivantes.

Dans le dosage d'α₂-AP par "STACHROM α₂-AP", on trouve une teneur d'α₂-AP inférieure à 5 % dans un plasma incubé en présence de venin et d'AMCHA ajoutés simultanément. Cette observation permet de conclure que l'AMCHA potentialise l'effet du venin sur l'inhibition de l'α₂-AP.

Dans le dosage de PAI, on trouve que le venin a un effet très important sur l'augmentation de la génération de plasmine et que cet effet est supérieur à celui de l'AMCHA (voir tableauxV,VI et VII ci-après). On constate que l'effet du venin est potentialisé par l'AMCHA en présence ou en l'absence de PAI (voir lesdits tableaux V, VI et VII).

Lorsque l'on utilise un plasma déficient en α₂-AP, la génération de plasmine est importante avec le venin, et elle l'est encore plus avec l'AMCHA. Ce résultat est lié à la transformation du Glu-plasminogène en Lys-plasminogène, et on constate que l'AMCHA dans ce cas ne potentialise pas le venin (voir tableau VIII ci-après).

### EXEMPLE 9

### - Effet du matériau métalloprotéinasique sur l'α₂-M -

On sait que les substances telles que l'EACA stimulent in vitro la génération de thrombine dans un plasma alors que l'effet de l'α₂-M est inhibé [cf. I.G.SLOAN et al., Thromb. Res., 44, pages 761-769, (1986)]. Ce phénomène peut être expliqué par le fait que les oméga-aminoacidestels que l'EACA et l'AMCHA possèdent un groupe amine terminal et sont capables d'inhiber l'α₂-AP par ce groupe amine terminal dès lors que des composés comme la méthylamine sont connus pour agir de cette façon.

On sait, d'autre part, qu'il a été signalé que certains venins perdent leur activité protéolytique quand ils sont incubés avec l'α₂-M. Mais on sait aussi que la complexation de l'α₂-M avec les protéinases de venin peut être retardée en présence de substrat de protéinases telles que l'α₂-M [voir l'article de L.F.KRESS précité].

Compte tenu de ces circonstances, on a voulu vérifier si oui ou non un matériau métalloprotéinasique tel qu'un venin (venin brut de B.arietans) en association le cas échéant avec un oméga-aminoacide (AMCHA) pouvait intervenir favorablement dans le dosage de PAI. Dans cette optique, on a mesuré la plasmine résiduelle après inhibition par l'aprotinine. On a observé que :
- l'aprotinine (0,1 TIU/ml) inhibe la plasmine purifiée mais pas le venin,
- la plasmine générée en présence d'aprotinine est environ de 3 % dans un système tamponné,
- la teneur en plasmine générée en milieu plasmatique et liée à l'α₂-M est inférieure à 5 %, même en l'absence d'α₂-AP.

Il en résulte que l'interférence de l'α₂-M est donc négligeable, même en l'absence d'α₂-AP.

Les résultats qui ont été obtenus sont consignés dans les tableaux IX et X ci-après. Le tableau IX est relatif à la mesure de la plasmine générée en présence d'urokinase (uPA), d'une part, en présence ou en l'absence d'aprotinine, d'autre part, dans un plasma normal en α₂-AP mais déplété en PAI. Le tableau X est relatif à la mesure de la plasmine générée en présence d'urokinase, d'une part, en présence ou en l'absence d'aprotinine, d'autre part, dans un plasma déplété en α₂-AP.

### EXEMPLE 10

### - Conditions optimales du dosage de tPA -

Les conditions optimales pour le dosage de tPA sont obtenues avec des concentrations de venin (de préférence venin brut de B.arietans tel que recueilli et fourni par les fermes d'élevage) :
- supérieures à 0,0125 mg/ml dans le mélange réactionnel (c'est-à-dire à des concentrations supérieures à 0,025 mg/ml dans le tampon),
- et plus préférentiellement, de l'ordre de 0,025 mg/ml dans le mélange réactionnel (i.e. 0,050 mg/ml dans le tampon),
   quel que soit le lot de venin.

La génération de plasmine optimale est obtenue en 2,50 h pour une gamme de tPA allant de 0 à 4 UI/ml, en tubes de verre ou de plastique. La courbe d'étalonnage obtenue est linéaire (voir courbe 8A de la figure 8). La courbe obtenue avec un plasma riche en tPA et dilué en plasma déplété en tPA et PAI est également linéaire (voir courbe 8B de la figure 8).

La figure 8 a été obtenue selon le système OD en ordonnées (à 405 nm)/tPA en UI/ml en abscisses.

Les résultats observés sur des plasmas obtenus après stase veineuse sont similaires, présence de venin, à ceux observés sur des euglobulines en l'absence de venin (voir tableau XII ci-après).

Avant stase veineuse, les résultats sont plus élevés sur les euglobulines car la mesure du tPA est évaluée avec les autres activateurs du plasminogène, surtout ceux de la voie dépendante du Facteur XII. Ces autres activateurs sont activés pendant la fabrication des euglobulines.

### EXEMPLE 11

### - Conditions optimales du dosage de PAI -

Les conditions optimales de dosage du PAI sont obtenues avec des concentrations :
- d'uPA de 10 à 50 UI/ml (de préférence 10 UI/ml), ou de tPA (selon le dosage au moyen de "STACHROM PAI"),
- de plasminogène de 1 à 4 U/ml (de préférence 3 U/ml) de mélange réactionnel, quel que soit le lot de plasminogène,
- d'AMCHA de 0,4 à 4 mmoles/ 1 (de préférence 0,8 mmole/l) de mélange réactionnel, ou de tout autre oméga-aminoacide,
- de venin (de préférence venin brut de B.arietans) de 0,1 à 1 mg/ml de plasma et mieux 0,24 mg/ml de plasma,
- de substrat (spécifique de la plasmine) de 1 à 5 micromoles/ml de plasma et mieux de 3,5 micromoles/ml de plasma.

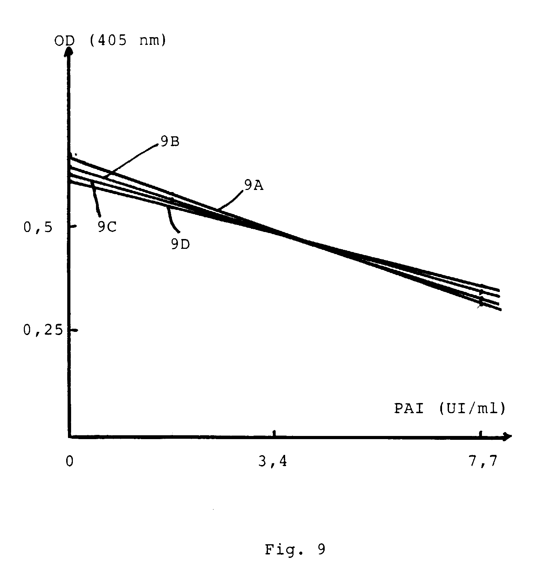

Dans ces conditions, la génération de plasmine optimale est obtenue en 3 minutes et l'hydrolyse du substrat est réalisée en 3 minutes. La courbe d'étalonnage réalisée avec un plasma déplété en PAI [plasma dans lequel la teneur en PAI est nulle (PAI = 0 UI/ml)] et un plasma riche en PAI (PAI = 7,7 UI/ml) est linéaire. Voir à cet effet la figure 9. Dans la figure 9, selon le système OD en ordonnées (à 405 nm)/PAI en UI/ml en abscisses, les droites 9A, 9B, 9C et 9D ont été obtenues avec respectivement quatre lots différents de venin de B. arietans.

Les résultats observés sur des plasmas normaux et des plasmas pathologiques (femmes enceintes et patients post-opératoires) sont similaires à ceux trouvés par la technique "STACHROM PAI". Les unités sont exprimées en UI/ml d'urokinase pour le PAI en présence de venin et en UI/ml de tPA pour le PAI par la technique "STACHROM PAI" (voir tableau XI). On constate toutefois une petite différence chez les femmes enceintes car le PAI-2 a une activité anti-uPA supérieure à l'activité anti-tPA (voir plasma F).

### UTILISATION DES REACTIFS

Pour la mise en oeuvre de l'invention, l'on recommande d'utiliser les réactifs selon les modalités suivantes :

### - Dosage de tPA

On utilisera :
- les réactifs de la trousse de dosage "STACHROM PA" de la société DIAGNOSTICA STAGO,
- un matériau métalloprotéinasique (constitué par du venin de serpent) lyophilisé seul ou lyophilisé avec le "STIMUGEN".

La reconstitution du flacon est donc identique à celle décrite dans la notice "STACHROM PA". Les excipients de lyophilisation du matériau métalloprotéinasique sont ceux décrits pour le "STIMUGEN".

### - Dosage de PAI

On utilisera :
- tPA : réactif de la trousse de dosage "STACHROM PAI" de la société DIAGNOSTICA STAGO,
- uPA (urokinase) : 20 UI/flacon, à reprendre par 2 ml d'eau distillée,
- excipients de lyophilisation : tampon phosphate (100 mM; pH 9,0) et BSA à 5 g/l,
- conservateur : gentamicine 50 mg/l,
- réactif, à reprendre par 2 ml d'eau distillée :
   - 1 volume de plasminogène purifié en tampon phosphate à pH 7,5,
   - 3 volumes de matériau métalloprotéinasique (venin de serpent) à la concentration de 0,15 mg/ml dans de l'eau distillée,
   - 2 volumes d'AMCHA à 4 mmoles/ l dans un diluant contenant un tampon phosphate (50 mM; pH 7,5), 3 % de glycine et 0,9 % de NaCl (conservateur : gentamicine à 50 mg/l),
- substrat : 16,5 micromoles/flacon à reprendre par 2 ml d'eau distillée, en excipient glycine classique,
- plasma étalon ou contrôle lyophilisé dans un milieu comprenant 3 % de glycine, 1 % de lactose, 1 % de saccharose et 50 mg/l de gentamicine.

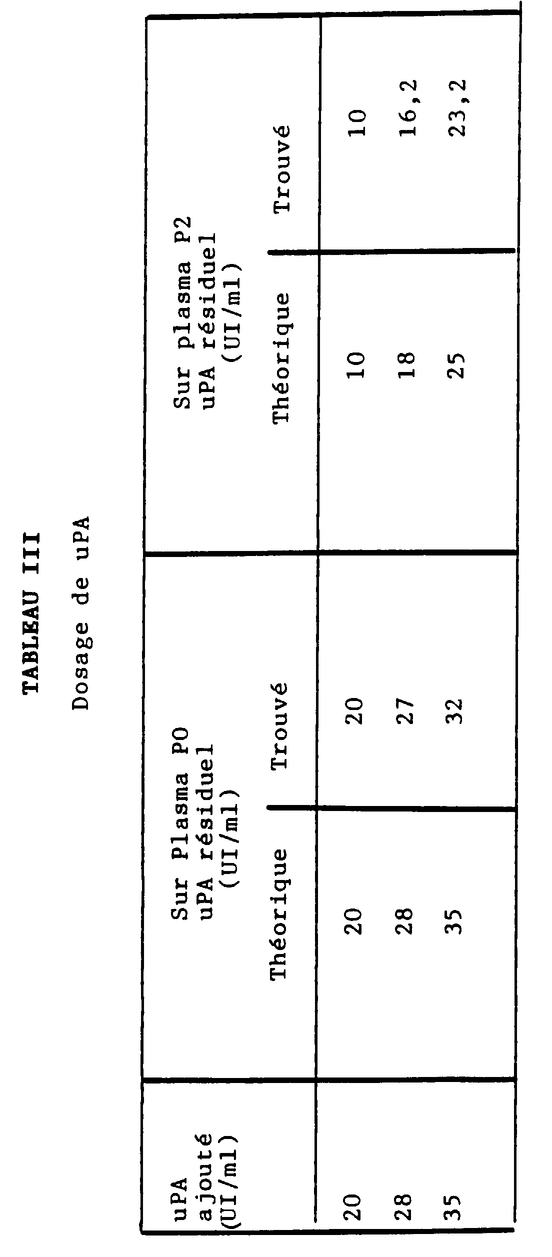

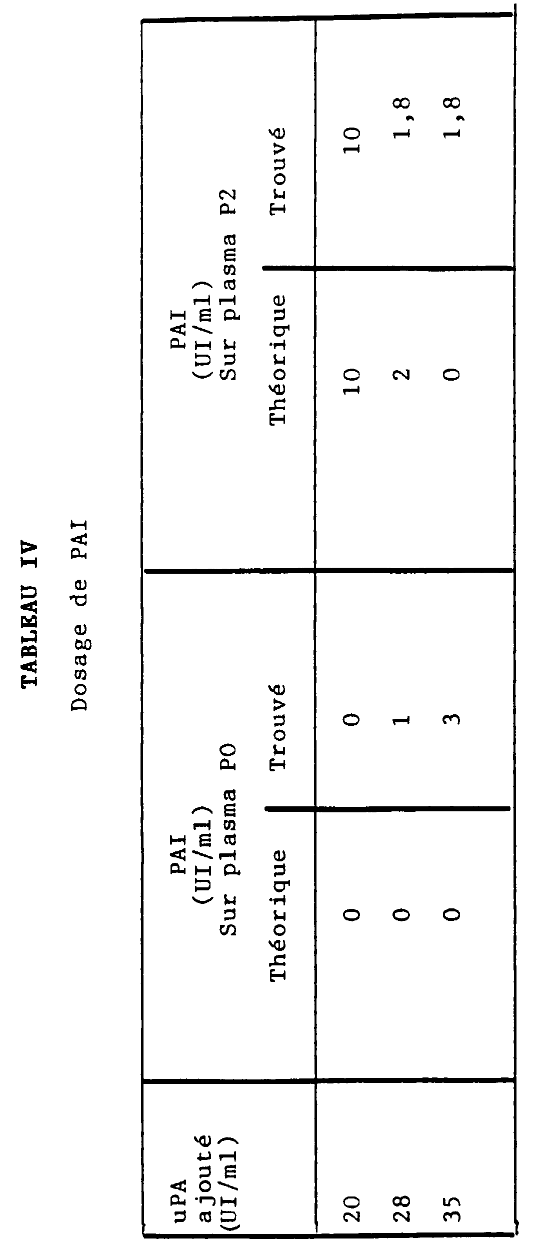

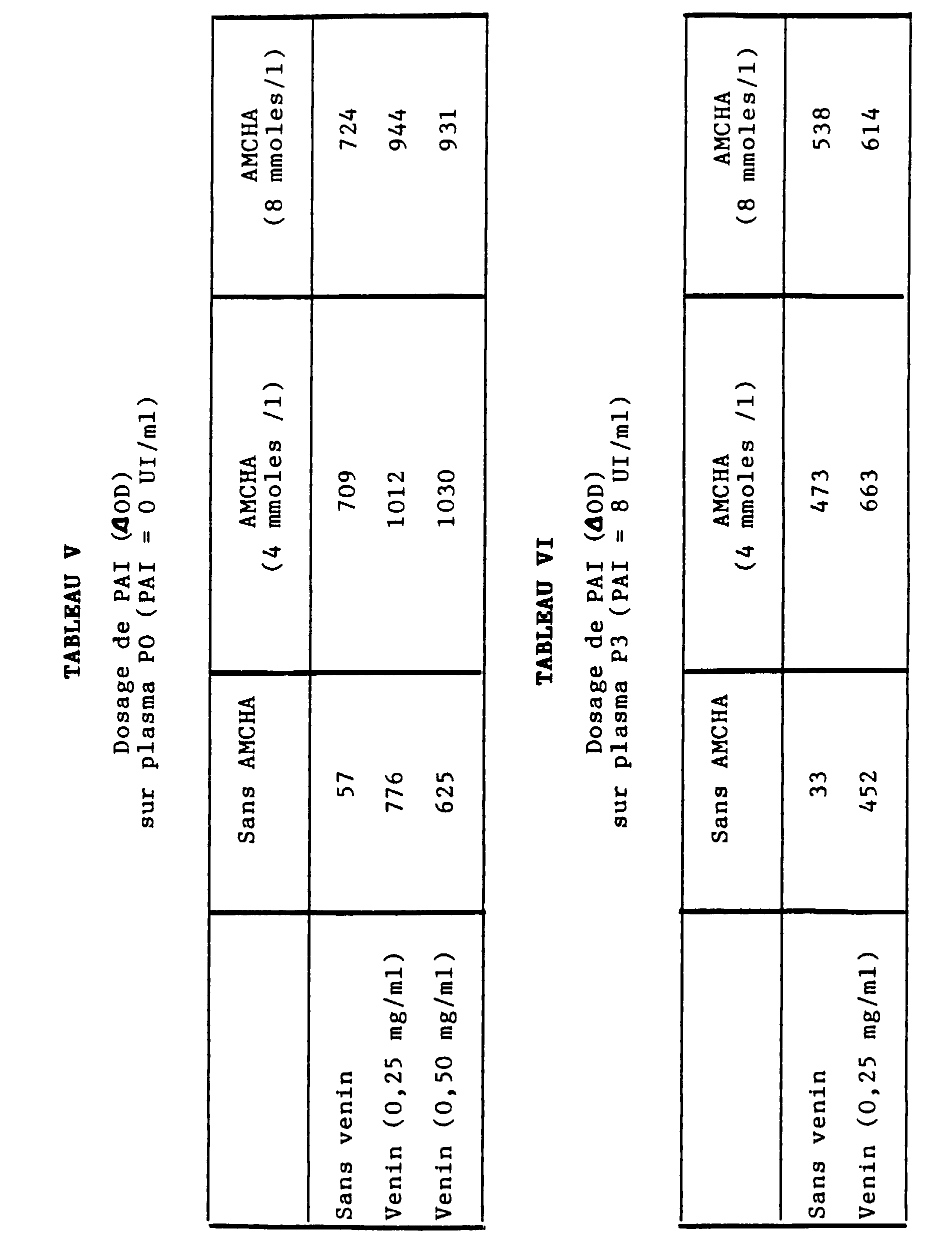

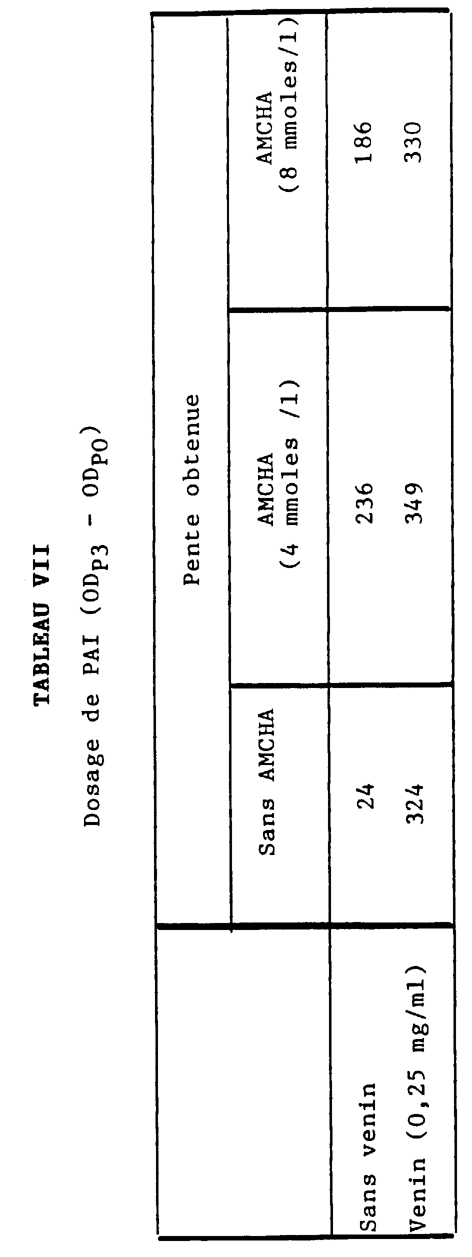

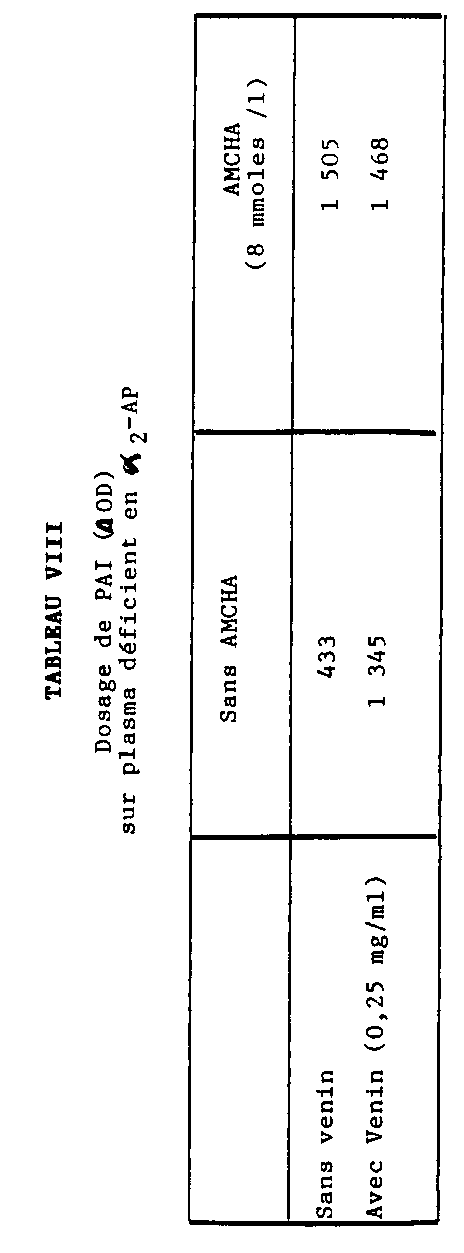

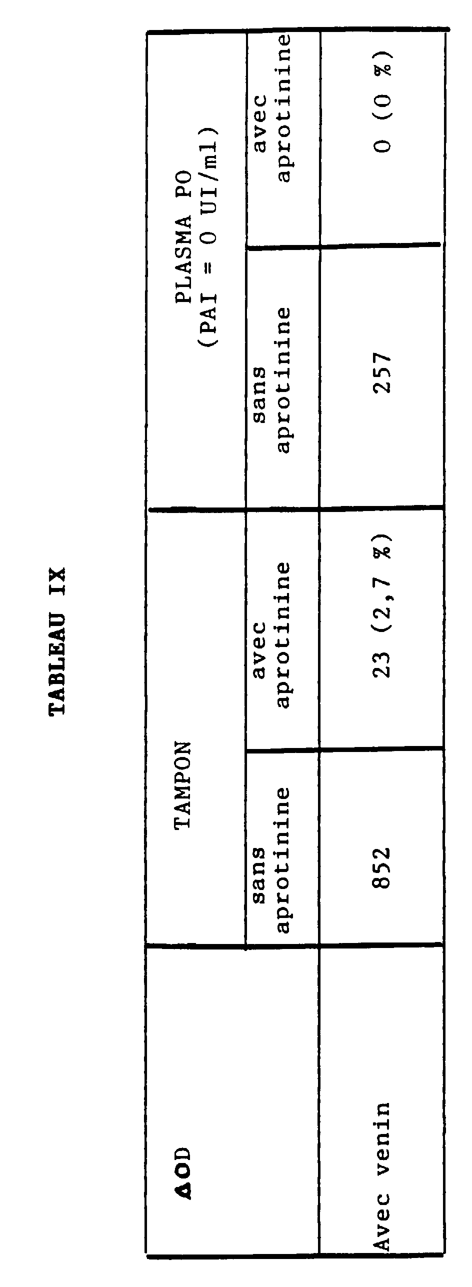

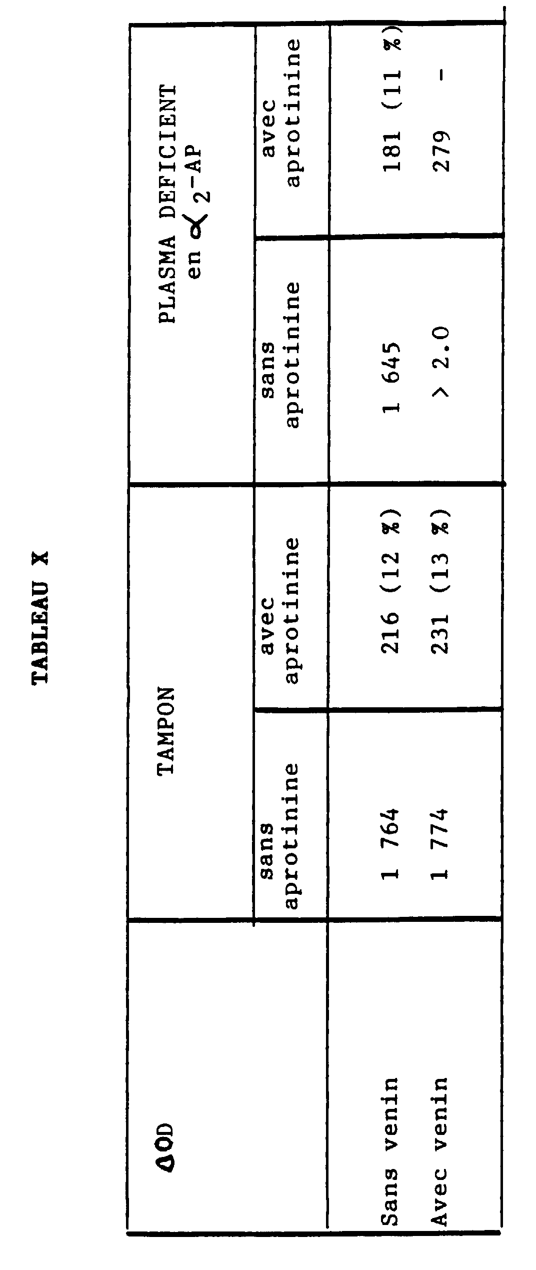

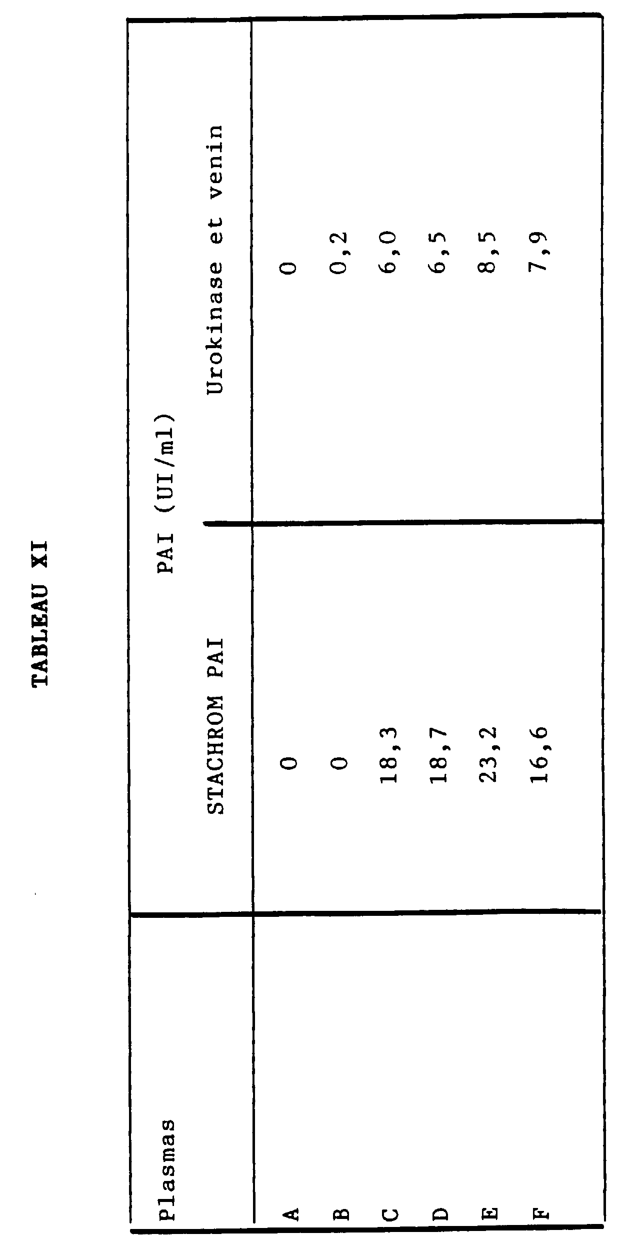

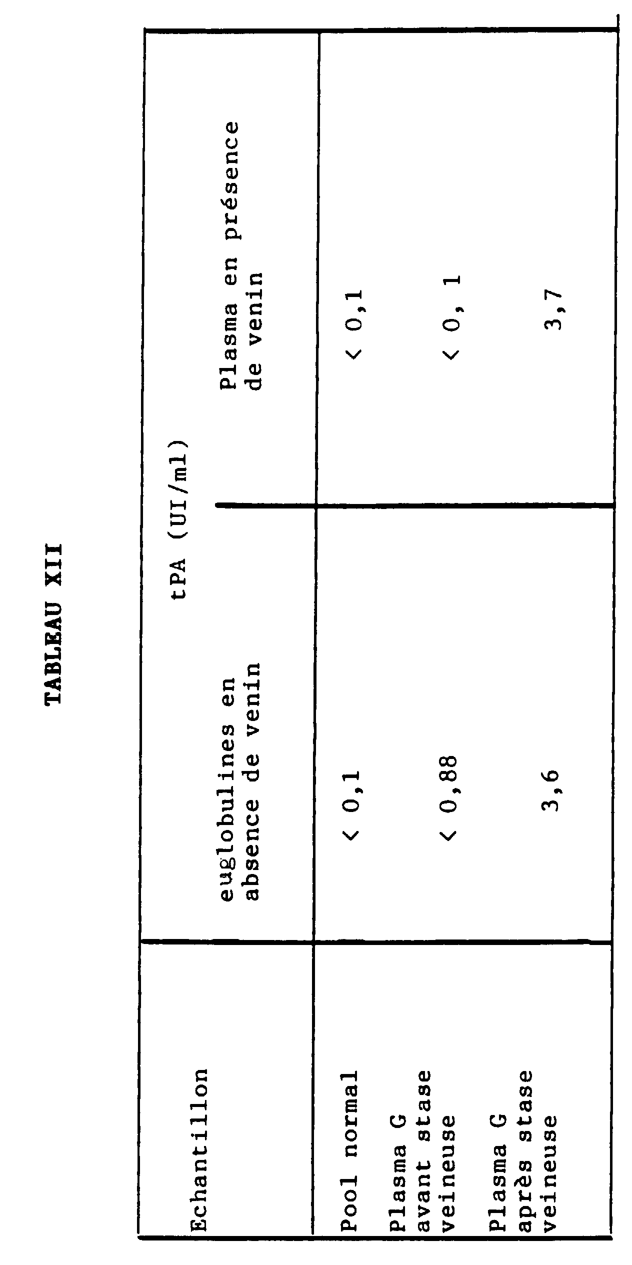

## Revendications

1. Procédé pour la détermination dans le plasma (i) d'un activateur du plasminogène choisi parmi l'ensemble constitué par les activateurs du plasminogène de type tissulaire (tPA) et les activateurs du plasminogène de type urokinase (uPA), et (ii) d'un inhibiteur (PAI) dudit activateur du plasminogène, ledit procédé, qui comporte l'inhibition des protéines plasmatiques α₂-antiplasmine et/ou α₂-macroglobuline au moyen d'une substance choisie parmi l'ensemble constitué par les matériaux métalloprotéinasiques, d'une part, et la transformation du plasminogène en plasmine puis le dosage de la plasmine résultant de ladite transformation, d'autre part, étant caractérisé en ce qu'il comprend (1) l'incorporation du matériau métalloprotéinasique dans le plasma à étudier, (2) l'ajout dans le milieu résultant d'un moyen stimulant la transformation du plasminogène en plasmine, puis (3) le dosage de la plasmine qui résulte de ladite transformation.

2. Procédé suivant la revendication 1, caractérisé en ce que pour la détermination du tPA, (a) l'on incorpore le matériau métalloprotéinasique dans le plasma à étudier dilué dans le tampon approprié, (b) incube à 37°C pendant 2 minutes, (c) ajoute un moyen stimulant la transformation du plasminogène en plasmine, (d) incube à 37°C pendant 150 minutes, (e) ajoute le substrat chromogène spécifique de la plasmine, puis (f) incube à 37°C pendant 5 minutes, la lecture de la densité optique correspondant à la libération du moyen chromogène étant ensuite effectuée notamment par comparaison avec un essai à blanc dans lequel le plasma à étudier additionné de matériau métalloprotéinasique du stade (a) est remplacé par un tampon additionné de matériau métalloprotéinasique.

3. Procédé suivant la revendication 2, dans lequel le matériau métalloprotéinasique est incorporé au stade (c) au lieu du stade (a).

4. Procédé suivant la revendication 1, caractérisé en ce que pour la détermination du PAI, (a) l'on incorpore tPA ou uPA dans le plasma à étudier, (b) incube à 37°C pendant 5 minutes, (c) ajoute un mélange constitué par du plasminogène, un ω-aminoacide et ledit matériau métalloprotéinasique, (d) incube à 37°C pendant 3 minutes, (e) ajoute le substrat chromogène spécifique de la plasmine, puis (f) incube à 37°C pendant 3 minutes, la lecture de la densité optique correspondant à la libération du moyen chromogène étant ensuite effectuée notamment par comparaison avec un essai à blanc dans lequel au stade (c) l'activateur du plasminogène (tPA ou uPA) est remplacé par un tampon.

5. Procédé suivant la revendication 2 ou 4, caractérisé en ce que le matériau métalloprotéinasique est choisi parmi les venins de Bitis arietans, Crotalus basiliscus et Lachesis muta.

6. Nécessaire pour la détermination d'un activateur du plasminogène de type tissulaire ou de type urokinase, d'une part, ou d'un inhibiteur d'activateur du plasminogène, d'autre part, caractérisé en ce qu'il comprend (A) au moins un matériau métalloprotéinasique choisi parmi l'ensemble constitué par les venins de Bitis arietans, Crotalus basiliscus et Lachesis muta, et (B) au moins un activateur du plasminogène choisi parmi l'ensemble constitué par les activateurs du plasminogène de type tissulaire (tPA) et les activateurs du plasminogène de type urokinase (uPA).

## Patentansprüche

1. Verfahren zur Bestimmung (i) eines Plasminogenaktivators, ausgewählt aus der Gruppe, bestehend aus den Plasminogenaktivatoren vom Gewebetyp (tPA) und den Plasminogenaktivatoren vom Urokinasetyp (uPA), und (ii) eines Hemmstoffes (PAI) dieses Plasminogenaktivators im Plasma, wobei das Verfahren einerseits die Hemmung der Plasmaproteine α₂-Antiplasmin und/oder α₂-Makroglobulin mittels einer Substanz, ausgewählt aus der Gruppe, bestehend aus Metalloproteinasestoffen, und andererseits die Umwandlung von Plasminogen in Plasmin und die Bestimmung des aus dieser Umwandlung entstandenen Plasmins beinhaltet und dadurch gekennzeichnet ist, daß es umfaßt: (1) die Zugabe des Metalloproteinasestoffes zu dem zu untersuchenden Plasma, (2) die Zugabe eines die Umwandlung von Plasminogen in Plasmin stimulierenden Mittels zu dem entstandenen Medium und danach (3) die Bestimmung des aus dieser Umwandlung entstandenen Plasmins.

2. Verfahren nach Anspruch 1, das zur Bestimmung von tPA gekennzeichnet ist durch (a) die Zugabe des Metalloproteinasestoffes zu dem zu untersuchenden, in einem geeigneten Puffer verdünnten Plasma, (b) Inkubieren bei 37°C für 2 Minuten, (c) Zugabe eines die Umwandlung von Plasminogen in Plasmin stimulierenden Mittels, (d) Inkubieren bei 37°C für 150 Minuten, (e) Zugabe eines für Plasmin spezifischen chromogenen Substrates und (f) Inkubieren bei 37°C für 5 Minuten, wobei das Ablesen der einer Freisetzung des chromogenen Mittels entsprechenden optischen Dichte dann insbesondere durch Vergleich mit einem Blindwert durchgeführt wird, bei dem das zu untersuchende mit dem Metalloproteinasestoff versetzte Plasma in Schritt (a) durch einen mit dem Metalloproteinasestoff versetzten Puffer ersetzt wird.

3. Verfahren nach Anspruch 2, bei dem der Metalloproteinasestoff in Schritt (c) anstatt in Schritt (a) zugegeben wird.

4. Verfahren nach Anspruch 1, das zur Bestimmung von PAI gekennzeichnet ist durch (a) die Zugabe von tPA oder uPA zu dem zu untersuchenden Plasma, (b) Inkubation für 5 Minuten bei 37°C, (c) Zugabe eines Gemisches aus Plasminogen, einer ω-Aminosäure und dem Metalloproteinasestoff, (d) Inkubation für 3 Minuten bei 37°C, (e) Zugabe des für Plasmin spezifischen chromogenen Substrates und (f) Inkubation für 3 Minuten bei 37°C, wobei das Ablesen der einer Freisetzung des chromogenen Mittels entsprechenden optischen Dichte dann insbesondere durch Vergleich mit einem Blindwert durchgeführt wird, bei dem in Schritt (c) der Plasminogenaktivator (tPA oder uPA) durch einen Puffer ersetzt ist.

5. Verfahren nach einem der Ansprüche 2 oder 4, dadurch gekennzeichnet, daß der Metalloproteinasestoff ausgewählt ist aus den Veninen von Bitis arietans, Crotalus basiliscus und Lachesis muta.

6. Kit zur Bestimmung eines Plasminogenaktivators vom Gewebetyp oder Urokinasetyp einerseits oder eines Plasminogenaktivator-Hemmstoffes andererseits, dadurch gekennzeichnet, daß es umfaßt (A) mindestens einen Metalloproteinasestoff, ausgewählt aus der Gruppe, bestehend aus den Veninen von Bitis arietans, Crotalus basiliscus und Lachesis muta, und (B) mindestens einen Plasminogenaktivator, ausgewählt aus der Gruppe, bestehend aus Plasminogenaktivatoren vom Gewebetyp (tPA) oder Plasminogenaktivatoren vom Urokinasetyp (uPA).

## Claims

1. A method of determining in plasma (i) a plasminogen activator selected from the group consisting of tissue plasminogen activators (tPA) and urokinase plasminogen activators (uPA), and (ii) an inhibitor (PAI) of said plasminogen activator, said method, which involves, on the one hand, inhibition of the plasma proteins α₂-antiplasmin and/or α₂-macroglobulin with a substance selected from the group consisting of metalloproteinase materials, and on the other, conversion of the plasminogen to plasmin then assaying the plasmin resulting from said conversion being characterized in that it comprises (1) incorporating the metalloproteinase material into the plasma to be tested, (2) adding a reagent stimulating the conversion of plasminogen to plasmin into the reaction medium thus obtained, then (3) measuring the plasmin resulting from said conversion.

2. A method according to claim 1, wherein, to determine tPA, (a) the metalloproteinase material is incorporated into the plasma to be studied, diluted in the appropriate buffer, (b) the mixture is incubated at 37°C for 2 minutes, (c) a reagent stimulating the conversion of plasminogen to plasmin is added, (d) the mixture is incubated at 37°C for 150 minutes, (e) the plasmin-specific chromogenic substrate is added, and then (f) the mixture is incubated at 37°C for 5 minutes, the reading of the optical density corresponding to the release of the chromogenic means subsequently being effected especially by comparison with a blank test in which the plasma to be studied, to which metalloproteinase material of stage (a) has been added, is replaced with a buffer to which metalloproteinase material has been added.

3. A method according to claim 2 in which the metalloproteinase material is incorporated at stage (c) instead of stage (a).

4. A method according to claim 1, wherein, to determine PAI, (a) tPA or uPA is incorporated into the plasma to be studied, (b) the mixture is incubated at 37°C for 5 minutes, (c) a mixture consisting of plasminogen, an ω-amino acid and said metalloproteinase material is added, (d) the mixture is incubated at 37°C for 3 minutes, (e) the plasmin-specific chromogenic substrate is added, and then (f) the mixture is incubated at 37°C for 3 minutes, the reading of the optical density corresponding to the release of the chromogenic means subsequently being effected especially by comparison with a blank test in which the plasminogen activator (tPA or uPA) is replaced with a buffer at stage (c).

5. A method according to claim 2 or 4, wherein the metalloproteinase material is selected from the venoms of ***Bitis arietans, Crotalus basiliscus* and *Lachesis muta.***

6. A kit for determining a tissue or urokinase plasminogen activator, on the one hand, or a plasminogen activator inhibitor, on the other, said kit comprising (A) at least one metalloproteinase material selected from the group consisting of the venoms of ***Bitis arietans, Crotalus basiliscus*** and ***Lachesis muta,*** and (B) at least one plasminogen activator selected from tPAs and uPAs.
